# FASCICULE DE BREVET EUROPEEN

(11) **EP 1 079 852 B1**
(45) Date de publication et mention de la délivrance du brevet: **04.10.2001**
(21) Numéro de dépôt: 99920904.2
(22) Date de dépôt: 21.05.1999
(51) Int. Cl.: A61K 38/44, A61K 31/52, A61K 31/355

(54) **COMPOSITIONS A BASE DE PENTOXIFYLLINE ET D' ANTICYTOKINE**
PENTOXIFYLLIN UND ANTIZYTOKIN ENTHALTENDE PHARMAZEUTISCHE ZUSAMMENSETZUNGEN
COMPOSITION BASED ON PENTOXIFYLLINE AND ANTICYTOKIN

(30) Priorité: 29.05.1998 FR 9806807
(43) Date de publication de la demande: 07.03.2001
(73) Titulaire: ASSISTANCE PUBLIQUE, HOPITAUX DE PARIS, 75004 Paris (FR); COMMISSARIAT A L'ENERGIE ATOMIQUE, 75015 Paris (FR)
(72) Inventeur: DELANIAN, Sylvie, F-75015 Paris (FR); LEFAIX, Jean-Louis, F-91640 Vaux Grigneuse (FR)
(74) Mandataire: Goulard, Sophie
(86) Numéro de dépôt international: FR9901208
(87) Numéro de publication internationale: WO9962543

(56) Documents cités:
- DATABASE MEDLINE [Online] US NATIONAL LIBRARY OF MEDICINE (NLM), BETHESDA, MD, US GOTTLOBER P ET AL: "Behandlung der kutanen Strahlenfibrose mit Pentoxifyllin und Vitamin E. Ein Erfahrungsbericht." XP002097529 & STRAHLENTHER ONKOL, JAN 1996, 172 (1) P34-8, GERMANY
- DELANIAN S. ET AL: "LA FIBROSE IATROGENIQUE EN CANCEROLOGIE (2E PARTIE): PRINCIPALES ETIOLOGIES ET POSSIBILITES THERAPEUTIQUES" BULLETIN DU CANCER (BULL. CANCER), 1993, 80/3 (202-212), XP002097528 France
- DATABASE EMBASE [Online] ELSEVIER SCIENCE PUBLISHERS, AMSTERDAM, NL WAXMAN K. ET AL: "Pentoxifylline alone versus pentoxifylline combined with superoxide dismutase prolongs survival in a rat hemorrhagic shock model" XP002097530 & RESUSCITATION (RESUSCITATION), 1993, 26/3 (237-242), Ireland

## Description

L'invention est relative à des compositions à base de pentoxifylline ainsi qu'à leur utilisation pour la préparation d'un médicament destiné à prévenir les fibroses localisées ou diffuses ou à traiter les dégénérescences conjonctivo-vasculaires.

Les dégénérescences conjonctivo-vasculaires sont des inflammations chroniques évoluées diffuses et interstitielles. Elles s'opposent aux inflammations aiguës, subaiguës, ou chroniques débutantes qui ne présentent pas encore de dégénérescence conjonctivo-vasculaire.

On considère généralement le développement d'une dégénérescence conjonctio-vasculaire comme la séquelle d'un phénomène déclenché initialement par un stress oxydatif, au niveau tissulaire. Les réactions précoces sont inflammatoires et ischémiques, associées occasionnellement à une perte de substance (nécrose ou ulcération). Plus tardivement après plusieurs étapes, la fibrose proprement dite s'installe et peut entraîner l'apparition de signes fonctionnels tels que la douleur. Ces étapes évolutives sont illustrées par exemple dans le développement de la fibrose radio-induite superficielle décrite ci-dessous. Au niveau cutané, on observe une déplétion cellulaire épidermique et une atrophie du derme, consistant en une diminution du nombre de fibroblastes et une hyalinisation du collagène. Au niveau sous-cutané, on observe, des modifications tissulaires, qui permettent de définir trois phases :
- une phase initiale pré-fibrotique, dans laquelle on observe une inflammation chronique aspécifique où la cellule endothéliale semble jouer un rôle prédominant ; ces modifications histologiques de type cicatriciel, liées à une atteinte combinée de l'endothélium et des cellules conjonctives, sont amplifiées par la sécrétion de facteurs de croissance, essentiellement du TNFα, qui induit la production de cytokines (IL-1, IL-6, IFN), augmente la production de radicaux libres, inhibe la synthèse de matrice extra-cellulaire et stimule sa dégradation ;
- une phase de fibrose constituée, dans laquelle on observe une accumulation de matrice extra-cellulaire, du fait de la sécrétion de facteur de croissance TGFβ, qui induit la prolifération des fibroblastes et des cellules endothéliales, la synthèse de matrice extra-cellulaire et inhibe la dégradation de cette dernière. La fibrose constituée peut être soit stable, soit aggravée et évoluer vers la sclérose des tissus.
- une phase tardive de sclérose, qui se traduit par une matrice extra-cellulaire paucicellulaire et peu vascularisée ainsi que par une diminution du taux de facteur de croissance TGFβ. Le délai d'apparition et la gravité de cette phase tardive de sclérose sont fonction du stress initial, de la physiologie globale de l'individu (nature du terrain, sexe, âge, pathologies aggravantes telles que des troubles métaboliques ou microcirculatoires) et de la localisation anatomique de l'agression. Cette étape peut évoluer vers la nécrose tissulaire lors d'une agression quelconque.

En d'autres termes, après une phase cellulaire non spécifique, la zone irradiée est le siège d'une prolifération endothéliale et fibroblastique intense, associée à des microthrombi fibrineux laissant apparaître des vaisseaux néoformés mal hiérarchisés et de nombreux shunts artério-veineux. Ultérieurement, la chute du débit sanguin local provoque un exsudat sérique avec dépôt de matrice qui aggrave l'ischémie. Au stade tardif des lésions constituées, le tissu fibreux est remodelé secondairement, la matrice augmentant sa densité et sa stabilité.

Au niveau cellulaire et moléculaire, le tissu fibreux est caractérisé par la présence persistante de myofibroblastes, qui prolifèrent et sécrètent les différents composants de la matrice extra-cellulaire sous l'impulsion des facteurs de croissance précités. La régulation quantitative du dépôt de matrice extra-cellulaire s'effectue au niveau de la synthèse post-transcriptionnelle du collagène : synthèse accrue de collagène de type I et de type III, de fibronectine, et d'acide hyaluronique, d'une baisse de l'activité des métalloprotéinases, d'une modification du rapport entre les divers isotypes de collagène et d'une sous-sulfatation des GAG (glycosaminoglycanes sulfatés).

Les modifications persistantes du programme de différenciation et de prolifération des cellules du tissu irradié sont mal connues. Le stress cellulaire provoqué par une irradiation se traduirait par une cascade d'interactions protéines-ADN avec l'activation de gènes dits précoces (*c-fos* et *c-jun*) qui seraient responsables de la réponse biologique ultérieure : réparation de l'ADN, arrêt de la croissance cellulaire ou sécrétion de cytokines (TNFα, PDGF ou IL1). Plus tardivement, la matrice extra-cellulaire qui abrite et relargue des facteurs de croissance à partir de récepteurs matriciels pourrait interférer avec ces phénomènes initiaux.

Dans les fibroses, les traitements habituellement proposés sont essentiellement symptomatiques et ne tiennent pas compte de manière systématique, de la phase de la maladie. De façon plus précise, les principales substances médicamenteuses testées en recherche pour traiter la pathologie fibreuse sont les anti-inflammatoires (corticoïdes, anti-inflammatoires non-stéroïdiens, colchicine, D-pénicillamine, antihistaminiques), les vasculotropes (inhibiteurs de l'enzyme de conversion, héparine, anti-agrégant plaquettaire, pentoxifylline), les anti-oxydants (malotilate, levamisole) et des molécules diverses (interféron, zinc,solenium, cuivre, acides aminés ...).

Parmi ces différentes thérapeutiques, la corticothérapie apparaît comme un traitement de référence contre l'inflammation. Mais si elle agit transitoirement sur les symptômes associés aux poussées inflammatoires, elle est relativement inefficace pour modifier l'évolution du processus. Il existe de plus un effet rebond régulier à l'arrêt du traitement.

Dans l'exemple des séquelles radio-induites, des résultats préliminaires intéressants ont été obtenus dans l'accélération de la cicatrisation des nécroses, avec la pentoxifylline (dérivé de la caféine), à raison de 1200 mg/jour pendant 3 à 6 mois (M.W. Dion et al., *Int. J. Radiation Oncology Biol. Phys.,* 1990, 19, 401-407 ; N.D. Futran et al., *The Laryngoscope,* 1997, 107, 391-395 ; M. Werner-Wasik et al., *Int. J. Radiation Oncology Biol. Phys.,* 1993, 25, 757-758 ; T.L. Cornelison et al., *Proc. Annu. Meet. Am. Assoc. Cancer Res.,* 1996, 37, A4185). Toutefois, à cette posologie, ce traitement présente l'inconvénient majeur d'entraîner de nombreux effets indésirables, principalement de nature digestive (nausée, diarrhée, brûlure gastrique) ou vasculaire ("flush" ou "rash" cutané), ou tels qu'une baisse de tension avec céphalée.

Les traitements par la pentoxifylline tels que décrits précédemment se limitent à améliorer le confort du patient en soulageant sa douleur (M. Werner-Wasik et al., précité ; T.L. Cornelison et al., précité) et en favorisant la cicatrisation de la nécrose tardive radio-induite (M.W. Dion et al).

L'α-tocophérol a également été proposé pour améliorer la symptomatologie des patients atteints de fibroses radio-induites (résumé paru dans Radiother. Oncol., 1997, 43, suppl. 1, S3). Les résultats obtenus sont superposables à ceux qui ont été obtenus avec la pentoxifylline.

En revanche, une association de 400 mg de pentoxifylline et de 500 UI d'α-tocophérol (vitamine E), en administration orale à raison de deux fois par jour, pendant 6 mois, permet de faire régresser une fibrose superficielle radio-induite, au stade de fibrose tardive constituée (J. Eur. Soc. Ther. Radiol. Oncol., 1996, 40, suppl. 1, S43, résumé 161). L'étude a été menée sur dix patients, qui ont tous présenté une amélioration fonctionnelle et mesurable.

Un traitement avec de la SOD (Cu/ZN SOD liposomiale), administrée par voie parentérale, a, par ailleurs, permis d'observer une réversibilité du processus fibreux et la régénération du tissu normal adjacent chez l'homme et le porc (S. Delanian et al., Radiother. Oncol., 1994, 32, 12-20 et JL Lefaix et al., Int. J. Radiation Oncol. Biol. Phys., 1996, 35, 2, 305-312).

En ce qui concerne les dégénérescences conjonctivo-vasculaires, qui correspondent à des inflammations chroniques évoluées diffuses, elles sont déclenchées par une agression de quelconque origine d'un tissu ou d'un organe (tissu sous-cutané, foie, poumon, rein, cerveau,...), par un stress oxydatif tissulaire. On peut citer par exemple un stress d'origine infectieuse (bactérienne, virale, parasitaire), chimique (toxique, médicamenteux), physique (chirurgie, ischémie,..), physique (UV, électrique, thermique,...).

Ce stress oxydatif entraîne par définition la production de radicaux libres, quels que soient la pathologie et l'organe concernés. L'inflammation peut évoluer (phases aiguë, subaiguë et chronique) vers une dégénérescence conjonctivo-vasculaire, si elle n'est pas convenablement soignée, ou si elle résiste à un traitement anti-inflammatoire.

En cas de palier d'aggravation supplémentaire, l'inflammation chronique évolue vers un stade pré-fibrotique, dans les termes définis ci-dessus et qui est caractérisé par une inflammation chronique aspécifique où la cellule endothéliale semble jouer un rôle prédominant, étape ultime avant qu'une phase de fibrose constituée ne se déclare. Cette dernière correspond histologiquement à une accumulation de matrice extra-cellulaire, de facteur TGFβ et à une prolifération fibroblastique intense. Elle peut être soit stable, soit aggravée et évoluer vers la sclérose voire nécrose des tissus, stades à partir desquels toute régénération tissulaire devient impossible et qui se traduit par une matrice extra-cellulaire paucicellulaire et peu vascularisée ainsi que par une diminution du taux de facteur de croissance TGFβ.

Ces inflammations apparaissent dans de nombreuses pathologies médicales, par exemple en cardiologie (athérosclérose, artérite, anévrisme artériel), en pneumologie (fibrose pulmonaire interstitielle chronique), en pathologie digestive (cirrhose hépatique, maladie de Crohn), en rhumatologie (polyarthrite rhumatoïde) ou encore en neurologie, en gynécologie ou en ophtalmologie.

Les traitements actuels sont également essentiellement symptomatiques (anti-inflammatoires essentiellement) et monospécifiques vis-à-vis de l'organe atteint : dialyse pour les reins, corticoïdes ou antiviraux pour le foie, oxygénothérapie hyperbare ou bronchodilatateurs pour les poumons...

Ainsi, il ressort de l'analyse de l'art antérieur que l'approche thérapeutique actuelle se borne à diriger le traitement pour palier le déficit fonctionnel de l'organe atteint, à un stade où la fibrose est déjà cliniquement déclarée. Les traitements utilisés en clinique sont spécifiques vis-à-vis de la pathologie en cause et ne peuvent pas être extrapolés à une utilisation préventive.

Par conséquent, les Inventeurs se sont donné pour but de pourvoir à des compositions pour la préparation d'un médicament destiné à prévenir toutes les fibroses, ainsi qu'à traiter plus particulièrement les dégénérescences conjonctivo-vasculaires, qui répondent mieux aux besoins de la pratique, notamment en ce qu'elles :
- sont effectivement efficaces à l'encontre des inflammations diffuses interstitielles,
- permettent une action curative, mais aussi préventive,
- sont applicables quel que soit l'organe atteint et
- agissent quel que soit le stade de la maladie.

Les Inventeurs ont en effet trouvé, de manière inattendue, que les échecs rencontrés tant dans la prévention des fibroses que dans le traitement des dégénérescences conjonctivo-vasculaires étaient dus au fait que l'on ne mettait en oeuvre que des traitements symptomatiques et que l'on ne tenait pas compte du stade de la maladie.

L'identification et la caractérisation physiopathologique du processus fibreux permettent maintenant d'envisager plusieurs niveaux de régulation ou de contrôle : métabolisme du collagène, prolifération fibroblastique et interactions cellules-matrice extra-cellulaire.

Les Inventeurs ont ainsi trouvé que :
- le choix thérapeutique doit être fixé par le stade d'évolution de la fibrose et que ce n'est que dans ces conditions que l'on peut obtenir une action de prévention dans l'ensemble des fibroses ou une régression substantielle et même une réversibilité totale de la dégénérescence conjonctivo-vasculaire et qu'en outre
- certaines associations médicamenteuses peuvent agir, de manière efficace, à n'importe quel stade de la dégénérescence conjonctivo-vasculaire aussi bien que dans la prévention de l'ensemble des fibroses.

Dans les conditions préconisées dans la présente invention, lorsque la lésion est jeune et inflammatoire (phase de constitution), on pourra prévenir la survenue d'une sclérose ; lorsque la lésion est très ancienne et constituée (phase tardive), on pourra tenter de réduire le bloc fibreux et même dans certains cas, le faire disparaître.

L'invention a pour objet l'utilisation d'une composition synergique comprenant essentiellement de la pentoxifylline à une dose unitaire de 100 à 400 mg et au moins un composé à action anti-cytokine à une dose unitaire comprise entre 1 000 et 3 000 UI et/ou de l'α-tocophérol à une dose unitaire de 100 à 500 UI, pour la préparation d'un médicament destiné à être utilisé dans la prévention des fibroses ou des dégénérescences conjonctivo-vasculaires, par l'administration orale de ladite composition, au plus tard au stade pré-fibrotique, pendant au moins 3 à 18 mois, à raison d'au plus deux fois/jour.

L'invention a également pour objet l'utilisation d'une composition synergique comprenant essentiellement de la pentoxifylline à une dose unitaire de 100 à 400 mg et au moins un composé à action anti-cytokine à une dose unitaire comprise entre 1 000 et 3 000 UI et/ou de l'α-tocophérol à une dose unitaire de 100 à 500 UI, pour la préparation d'un médicament destiné à être utilisé dans le traitement des dégénérescences conjonctivo-vasculaires, par l'administration orale, en particulier lors de la phase de fibrose en phase de constitution, pendant au moins 3 à 18 mois, à raison d'au plus deux fois/jour.

Selon une disposition avantageuse de la présente invention, le composé à action anti-cytokine est une SOD.

Les SOD (superoxyde dismutases) ont été caractérisées en 1968 par McCord et Fridovich (J. Biol. Chem., 1969, 244, 6049-6055). Ces enzymes favorisent l'élimination du radical superoxyde (O₂⁻) par dismutation et constituent donc un système de protection contre les effets délétères de ce radical, susceptible de se former in vivo, à partir de l'oxygène tissulaire. L'utilisation des SOD a donc été préconisée dans les affections qui impliquent les radicaux libres, donc dans les processus inflammatoires, notamment les rhumatismes et les fibroses.

Ladite SOD est de préférence une SOD hétérologue, notamment une SOD d'origine végétale ou animale obtenue par extraction ou recombinaison génétique.

Des formulations pouvant comprendre une SOD administrable par voie orale sont plus particulièrement décrites dans la Demande de Brevet Européen n° 0 804 225.

Selon une autre disposition avantageuse de l'invention, lorsque ladite composition comprend de la pentoxifylline et de l'α-tocophérol, aux doses précitées, elle peut en outre comprendre un diphosphonate à une dose unitaire comprise entre 400 et 800 mg.

Le diphosphonate est notamment sélectionné dans le groupe constitué par l'acide étidronique, l'acide tiludronique, l'acide clodronique et leurs sels.

Avantageusement, la composition adaptée à l'utilisation selon la présente invention comprend de la pentoxifylline et de l'α-tocophérol, ou bien de la pentoxifylline et une SOD, ou encore de la pentoxifylline, une SOD et de l'α-tocophérol, ou encore de la pentoxifylline, de l'α-tocophérol et un diphosphonate, administrés aux doses précitées.

De manière avantageuse, les différents produits inclus dans les compositions synergiques selon l'invention peuvent se présenter sous la forme d'une préparation combinée pour une utilisation simultanée, séparée ou séquentielle dans le traitement des fibroses localisées ou diffuses ou les dégénérescences conjonctivo-vasculaires.

Ces quantités sont adaptées à un adulte dont le poids est d'environ 60 kg.

De façon inattendue, l'utilisation d'une telle composition synergique permet non seulement de traiter une dégénérescence conjonctivo-vasculaire, mais aussi de prévenir son apparition. Il y aura prévention lorsque la composition sera utilisée au stade de l'inflammation chronique : phase pré-fibrotique, apparaissant souvent dans les 6 mois suivant une agression et il y aura traitement lorsque l'utilisation interviendra lors de la phase de fibrose constituée ou de la phase de sclérose (soit plutôt après 6 mois suivant l'agression initiale, après le déclenchement de l'inflammation).

De manière surprenante, une telle utilisation permet d'aboutir à la réversibilité de la fibrose constituée, c'est-à-dire à sa disparition définitive.

Selon une disposition avantageuse de la présente invention, l'utilisation est caractérisée en ce que ladite composition comprend de la pentoxifylline à une dose unitaire de 400 mg et de l'α-tocophérol à une dose unitaire de 500 UI.

Selon une autre disposition avantageuse de la présente invention, l'utilisation est caractérisée en ce que ladite composition comprend de la pentoxifylline à une dose unitaire de 400 mg et une SOD à une dose unitaire de 1500 UI.

Selon encore une autre disposition avantageuse de l'invention, l'utilisation est caractérisée en ce que ladite composition comprend de la pentoxifylline à une dose unitaire de 100 à 400 mg, une SOD à une dose unitaire de 1 000 3 000 UI et de l'α-tocophérol à une dose unitaire de 100 à 500 UI.

Selon encore une autre disposition avantageuse de la présente invention, l'utilisation est caractérisée en ce que ladite composition comprend de la pentoxifylline à une dose unitaire de 400 mg, de l'α-tocophérol à une dose unitaire de 100 à 500 UI et un diphosphonate à une dose unitaire de 400 à 800 mg.

Ainsi, l'utilisation selon l'invention d'une composition telle que décrite ci-dessus permet de diminuer la dose nécessaire de pentoxifylline (800 mg/jour), par rapport aux compositions de l'art antérieur utilisant de la pentoxifylline (1200 mg/jour). Ceci provient d'un effet de synergie entre la pentoxifylline et le composé à action anti-cytokine et/ou l'α-tocophérol.

La diminution de la dose nécessaire de pentoxifylline permet de minimiser les effets secondaires indésirables de celle-ci.

De manière générale, la combinaison de ces différents produits présente une action de synergie.

La présente invention est relative à une composition synergique comprenant essentiellement de la pentoxifylline et au moins un composé à action anti-cytokine, ainsi qu'une composition comprenant essentiellement de la pentoxifylline, au moins un composé à action anti-cytokine et de l'α-tocophérol, ainsi qu'une composition comprenant essentiellement de la pentoxifylline, de l'α-tocophérol et un diphosphonate.

De telles compositions peuvent avantageusement être utilisées dans la prévention de l'ensemble des fibroses (administration au stade pré-fibrotique ou même avant) ainsi que dans le traitement des dégénérescences conjonctivo-vasculaires.

Comme précisé ci-dessus, les différents produits les composant peuvent être administrés simultanément, séparément ou séquentiellement.

De manière avantageuse, le composé à action anti-cytokine est une SOD, telle que définie ci-dessus.

Selon un mode de réalisation préféré de ladite composition, elle comprend de 100 à 400 mg de pentoxifylline et de 1000 à 3000 UI de SOD, ou bien de 100 à 400 mg de pentoxifylline, de 1000 à 3000 UI de SOD et de 100 à 500 UI d'α-tocophérol ou bien de 100 à 400 mg de pentoxifylline, de 100 à 500 UI d'α-tocophérol et de 400 à 800 mg d'un diphosphonate.

Lesdites compositions sont avantageusement associées à au moins un véhicule pharmaceutiquement acceptable.

Outre les dispositions qui précèdent, l'invention comprend encore d'autres dispositions qui ressortiront de la description qui va suivre, qui se réfère à un exemple de mise en oeuvre de la présente invention.

Il doit être bien entendu, toutefois, que cet exemple est donné uniquement à titre d'illustration de l'objet de l'invention, dont il ne constitue en aucune manière une limitation.

### EXEMPLE 1 : Etude in vivo de l'effet anti-fibrotique de l'association pentoxifylline/α-tocophérol, chez des porcs atteints de fibrose pulmonaire (dégénérescence conjonctivo-vasculaire).

### - Méthode

Trois groupes de cinq porcs d'environ 120 kg présentant une fibrose pulmonaire depuis six mois ont été utilisés.

Un premier groupe de cinq porcs a reçu une dose quotidienne de 1600 mg de pentoxifylline, par voie orale, pendant six mois.

Un deuxième groupe de cinq porcs a reçu, pendant la même période, une dose quotidienne de 1600 mg de pentoxifylline associée à 2000 UI d'α-tocophérol, par voie orale. On remarquera que ces doses sont le double de celles préconisées pour un humain, étant donné le poids de 120 kg des porcs testés.

Le troisième groupe sert de contrôle (les porcs sont nourris avec de la nourriture normale, sans ajout de pentoxifylline ni d'α-tocophérol).

Après six mois de traitement, les porcs sont autopsiés et un examen histologique des tissus pulmonaires atteints est réalisé.

### - Tolérance

Aucun changement n'a été observé dans le comportement des deux groupes de porcs pendant la durée du traitement, ni aucun signe clinique ou anatomique anormal.

### - Evolution quantitative :

Par comparaison avec le groupe de contrôle, la fibrose pulmonaire n'a pas régressé chez les porcs traités avec la pentoxifylline seule, mais elle a régressé de façon significative chez les porcs traités par l'association pentoxifylline/α-tocophérol, après six mois de traitement.

Par rapport au groupe de contrôle, le volume de la tumeur fibrotique est similaire pour le groupe traité par pentoxifylline, alors qu'il est significativement plus faible pour le groupe traité par l'association pentoxifylline/α-tocophérol

### - Observations immunohistologiques, facteurs TNFα et TGFβ-1

### . Protocole opératoire

Des échantillons tissulaires sont placés dans un tampon de formaline à 10% et immobilisés dans de la cire de paraffine. Des coupes de 5 µm sont préparées, puis la cire est éliminée et les coupes sont soit colorées avec une solution d'hématoxiline-éosine-safranine, soit perméabilisées avec une solution de Triton à 1 % pour un marquage par immunofluorescence. Après la perméabilisation, les coupes sont lavées dans du PBS, saturées avec une solution de BSA à 2 % dans du PBS, puis incubées pendant une nuit à 4°C avec l'anticorps primaire. Les coupes sont ensuite lavées trois fois dans du PBS, incubées pendant 45 minutes avec l'anticorps secondaire, mélangées volume à volume avec du PBS-glycérol et examinées à l'aide d'un microscope à épifluorescence BH2 Olympus.

Chaque anticorps est testé pour obtenir la concentration qui donne la fluorescence spécifique maximale et la fluorescence de fond minimale. L'anticorps primaire anti-TNFα, un anticorps polyclonal dirigé contre le TNFα humain recombinant purifié (Genzyme Diagnostics), est utilisé à des dilutions allant de 1/100 à 1/250. Une dilution de 1/150 est utilisée pour le grossissement. L'anticorps primaire anti-TGFβ-1, un anticorps polyclonal dirigé contre un peptide correspondant aux acides aminés 328-353 de la région carboxy-terminale du TGFβ-1 humain (Santa Cruz Biotechnology, Inc), est utilisé à des dilutions allant de 1/50 à 1/150. Une dilution de 1/50 est utilisée pour le grossissement. Le second anticorps (anticorps de lapin conjugué au FITC, Immunotech) est utilisé à une dilution de 1/100.

### . Résultats

Concernant le TNFα, son taux d'expression est équivalent pour le groupe de contrôle, pour le groupe traité à la PTX et pour le groupe traité par l'association PTX/α-tocophérol.

Le taux d'expression et la distribution cellulaire du TGFβ-1 sont comparables pour le groupe traité par la PTX et pour le groupe de contrôle.

En revanche, le taux d'expression du TGFβ-1 dans la matrice extracellulaire est plus faible chez les porcs traités par PTX/α-tocophérol que pour le groupe de contrôle. Un léger marquage anti-TGFβ-1 est détecté dans les fibroblastes et les cellules endothéliales. Les cellules inflammatoires résiduelles sont toujours marquées avec l'anticorps anti-TGFβ-1.

### EXEMPLE 2

Des résultats similaires sont obtenus avec des compositions comprenant de la pentoxifylline et de la SOD ou de la pentoxifylline, une SOD et de l'α-tocophérol.

### EXEMPLE 3

Des compositions comprenant 400 mg de pentoxifylline, 500 UI d'α-tocophérol et 1 600 mg de clodronate ont été administrées chez l'Homme.

On observe chez 7 patients présentant une ostéo-radionécrose, une amélioration nette après trois mois de traitement : réduction des douleurs, sensation de solidité, apparition de cal osseux.

## Revendications

1. Utilisation d'une composition synergique comprenant essentiellement de la pentoxifylline à une dose unitaire de 100 à 400 mg et au moins un composé à action anti-cytokine à une dose unitaire comprise entre 1 000 et 3 000 UI et/ou de l'α-tocophérol à une dose unitaire de 100 à 500 UI pour la préparation d'un médicament destiné à être utilisé dans la prévention des fibroses ou des dégénérescences conjonctivo-vasculaires, par l'administration orale de ladite composition, au plus tard au stade pré-fibrotique, pendant au moins 3 à 18 mois, à raison d'au plus deux fois/jour.

2. Utilisation d'une composition synergique comprenant essentiellement de la pentoxifylline à une dose unitaire de 100 à 400 mg et au moins un composé à action anti-cytokine à une dose unitaire comprise entre 1 000 et 3 000 UI et/ou de l'α-tocophérol à une dose unitaire de 100 à 500 UI, pour la préparation d'un médicament destiné à être utilisé dans le traitement des dégénérescences conjonctivo-vasculaires, par l'administration orale, pendant au moins 3 à 18 mois, à raison d'au plus deux fois/jour.

3. Utilisation selon la revendication 1 ou 2, **caractérisée en ce que** le composé à action anti-cytokine est une SOD.

4. Utilisation selon la revendication 1 ou la revendication 2, **caractérisée en ce que** lorsque ladite composition comprend de la pentoxifylline et de l'α-tocophérol, aux doses précitées, elle peut en outre comprendre un diphosphonate à une dose unitaire comprise entre 400 et 800 mg.

5. Utilisation selon l'une quelconque des revendications 1 à 4, **caractérisée en ce que** ladite composition comprend essentiellement de la pentoxifylline à une dose unitaire de 400 mg et de l'α-tocophérol à une dose unitaire de 500 UI.

6. Utilisation selon l'une quelconque des revendications 1 à 3, **caractérisée en ce que** ladite composition comprend de la pentoxifylline à une dose unitaire de 400 mg et une SOD à une dose unitaire de 1 500 UI.

7. Utilisation selon l'une quelconque des revendications 1 à 3, **caractérisée en ce que** ladite composition comprend de la pentoxifylline à une dose unitaire de 400 mg, une SOD à une dose unitaire de 1 500 UI et do l'α-tocophérol à une dose unitaire de 500 UI.

8. Utilisation selon l'une quelconque des revendications 1 à 4, **caractérisée en ce que** ladite composition comprend de la pentoxifylline à une dose unitaire de 100 à 400 mg, de l'α-tocophérol à une dose unitaire de 100 à 500 UI et un diphosphonate à une dose unitaire de 400 à 800 mg.

9. Composition synergique destinée à l'administration orale, **caractérisée en ce qu'**elle comprend essentiellement de la pentoxifylline à une dose unitaire de 100 à 400 mg et au moins un composé à action anti-cytokine à une dose unitaire comprise entre 1 000 et 3 000 UI.

10. Composition synergique destinée à l'administration orale, **caractérisée en ce qu'**elle comprend essentiellement de la pentoxifylline à une dose unitaire de 100 à 400 mg, au moins un composé à action anti-cytokine à une dose unitaire comprise entre 1 000 et 3 000 UI et de l'α-tocophérol à une dose unitaire de 100 à 500 UI.

11. Composition synergique destinée à l'administration orale, **caractérisée en ce qu'**elle comprend essentiellement de la pentoxifylline à une dose unitaire de 100 à 400 mg, de l'α-tocophérol à une dose unitaire de 100 à 500 Ul et un diphosphonate à une dose unitaire de 400 à 800 mg.

12. Composition selon l'une quelconque des revendications 9 à 11, pour son utilisation comme médicament.

13. Composition selon la revendication 9 ou la revendication 10, **caractérisée en ce que** le composé à action anti-cytokine est une SOD.

14. Produits synergiques contenant de la pentoxifylline, de l'α-tocophérol et soit un composé à action anti-cytokine ou un diphosphonate comme préparation combinée pour une utilisation simultanée, séparée ou séquentielle dans le traitement des fibroses localisées ou diffuses ou les dégénérescences conjonctivo-vasculaires.

## Patentansprüche

1. Verwendung einer synergetischen Zusammensetzung, umfassend im Wesentlichen Pentoxifyllin in einer Dosiseinheit von 100 bis 400 mg und mindestens eine Verbindung mit Anticytokinwirkung in einer Dosiseinheit von zwischen 1000 und 3000 IE und/oder α-Tocopherol in einer Dosiseinheit von 100 bis 500 IE, zur Herstellung eines Arzneimittels, das dazu bestimmt ist, bei der Prävention von Fibrosen oder Degenerationserscheinungen in Verbindung mit Gefäßen durch orale Verabreichung der Zusammensetzung spätestens im Vor-Fibrosestadium über 3 bis 18 Monate höchstens zeimal/Tag verwendet zu werden.

2. Verwendung einer synergetischen Zusammensetzung, umfassend im Wesentlichen Pentoxifyllin in einer Dosiseinheit von 100 bis 400 mg und mindestens eine Verbindung mit Anticytokinwirkung in einer Dosiseinheit von zwischen 1000 und 3000 IE und/oder α-Tocopherol in einer Dosiseinheit von 100 bis 500 IE, zur Herstellung eines Arzneimittels, das dazu bestimmt ist, in der Behandlung von Degenerationserscheinungen in Verbindung mit Gefäßen durch orale Verabreichung über 3 bis 18 Monate höchstens zweimal/Tag verwendet zu werden.

3. Verwendung nach Anspruch 1 oder 2, **dadurch gekennzeichnet, dass** die Verbindung mit Anticytokinwirkung eine SOD ist.

4. Verwendung nach Anspruch 1 oder Anspruch 2, **dadurch gekennzeichnet , dass** die Zusammensetzung, die Pentoxifyllin und α-Tocopherol in den vorstehend angegebenen Dosen umfasst, außerdem ein Diphosphonat in einer Dosiseinheit von zwischen 400 und 800 mg umfassen kann.

5. Verwendung nach einem der vorhergehenden Ansprüche 1 bis 4, **dadurch gekennzeichnet , dass** die Zusammensetzung im Wesentlichen Pentoxifyllin in einer Dosiseinheit von 400 mg und α-Tocopherol in einer Dosiseinheit von 500 IE umfasst.

6. Verwendung nach einem der Ansprüche 1 bis 3, **dadurch gekennzeichnet, dass** die Zusammensetzung Pentoxifyllin in einer Dosiseinheit von 400 mg und eine SOD in einer Dosiseinheit von 1500 IE umfasst.

7. Verwendung nach einem der Ansprüche 1 bis 3, **dadurch gekennzeichnet , dass** die Zusammensetzung Pentoxifyllin in einer Dosiseinheit von 400 mg, eine SOD in einer Dosiseinheit von 1500 IE und α-Tocopherol in einer Dosiseinheit von 500 IE umfasst.

8. Verwendung nach einem der Ansprüche 1 bis 4, **dadurch gekennzeichnet , dass** die Zusammensetzung Pentoxifyllin in einer Dosiseinheit von 100 bis 400 mg, α-Tocopherol in einer Dosiseinheit von 100 bis 500 IE und ein Diphosphonat in einer Dosiseinheit von 400 bis 800 mg umfasst.

9. Synergetische Zusammensetzung, die zur oralen Verabreichung bestimmt ist, **dadurch gekennzeichnet, dass** sie im Wesentlichen Pentoxifyllin in einer Dosiseinheit von 100 bis 400 mg und mindestens eine Verbindung mit Anticytokinwirkung in einer Dosiseinheit von zwischen 1000 und 3000 IE umfasst.

10. Synergetische Zusammensetzung, die zur oralen Verabreichung bestimmt ist, **dadurch gekennzeichnet, dass** sie im Wesentlichen Pentoxifyllin in einer Dosiseinheit von 100 bis 400 mg, mindestens eine Verbindung mit Anticytokinwirkung in einer Dosiseinheit von zwischen 1000 und 3000 IE und α-Tocopherol in einer Dosiseinheit von 100 bis 500 IE umfasst.

11. Synergetische Zusammensetzung, die zur oralen Verabreichung bestimmt ist, **dadurch gekennzeichnet, dass** sie im Wesentlichen Pentoxifyllin in einer Dosiseinheit von 100 bis 400 mg, α-Tocopherol in einer Dosiseinheit von 100 bis 500 IE und ein Diphosphonat in einer Dosiseinheit von 400 bis 800 mg umfasst.

12. Zusammensetzung nach einem der Ansprüche 9 bis 11 zur Verwendung als Arzneimittel.

13. Zusammensetzung nach Anspruch 9 oder 10, **dadurch gekennzeichnet , dass** die Verbindung mit Anticytokinwirkung eine SOD ist.

14. Synergetische Produkte, die Pentoxifyllin, α-Tocopherol und entweder eine Verbindung mit Anticytokinwirkung oder ein Diphosphonat enthalten, als Kombinationspräparat zur gleichzeitigen, getrennten oder aufeinander folgenden Verwendung in der Behandlung von lokalisierten oder diffusen Fibrosen oder Degenerationserscheinungen in Verbindung mit Gefäßen.

## Claims

1. Use of a synergistic composition comprising essentially pentoxifylline, at a unit dose of 100 to 400 mg, and at least one compound with anticytokine 5 action, at a unit dose of between 1000 and 3000 IU, and/or α-tocopherol, at a unit dose of 100 to 500 IU, for preparing a medicinal product intended to be used in the prevention of fibrosis or of connective tissue/vascular tissue degeneration, by oral administration of said composition, at the latest at the prefibrotic stage, for at least 3 to 18 months, no more than twice/day.

2. Use of a synergistic composition comprising essentially pentoxifylline, at a unit dose of 100 to 400 mg, and at least one compound with anticytokine action, at a unit dose of between 1000 and 3000 IU, and/or α-tocopherol, at a unit dose of 100 to 500 IU, for preparing a medicinal product intended to be used in the treatment of connective tissue/vascular tissue degeneration, by oral administration, for at least 3 to 18 months, no more than twice/day.

3. Use according to either of claims 1 and 2, **characterized in that** the compound with anticytokine action is an SOD.

4. Use according to either claim 1 or claim 2, **characterized in that** when said composition comprises pentoxifylline and α-tocopherol, at the abovementioned doses, it can also comprise a diphosphonate at a unit dose of between 400 and 800 mg.

5. Use according to any one of claims 1 to 4, **characterized in that** said composition comprises essentially pentoxifylline at a unit dose of 400 mg and α-tocopherol at a unit dose of 500 IU.

6. Use according to any one of claims 1 to 3, **characterized in that** said composition comprises pentoxifylline at a unit dose of 400 mg and an SOD at a unit dose of 1500 IU.

7. Use according to any one of claims 1 to 3, **characterized in that** said composition comprises pentoxifylline at a unit dose of 400 mg, an SOD at a unit dose of 1500 IU and α-tocopherol at a unit dose of 500 IU.

8. Use according to any one of claims 1 to 4, **characterized in that** said composition comprises pentoxifylline at a unit dose of 100 to 400 mg, α-tocopherol at a unit dose of 100 to 500 IU and a diphosphonate at a unit dose of 400 to 800 mg.

9. Synergistic composition intended for oral administration, **characterized in that** it comprises essentially pentoxifylline at a unit dose of 100 to 400 mg and at least one compound with anticytokine action at a unit dose of between 1000 and 3000 IU.

10. Synergistic composition intended for oral administration, **characterized in that** it comprises essentially pentoxifylline at a unit dose of 100 to 400 mg, at least one compound with anticytokine action at a unit dose of between 1000 and 3000 IU and α-tocopherol at a unit dose of 100 to 500 IU.

11. Synergistic composition intended for oral administration, **characterized in that** it comprises essentially pentoxifylline at a unit dose of 100 to 400 mg, α-tocopherol at a unit dose of 100 to 500 IU and a diphosphonate at a unit dose of 400 to 800 mg.

12. Composition according to any one of claims 9 to 11, for its use as a medicinal product.

13. Composition according to either claim 9 or claim 10, **characterized in that** the compound with anticytokine action is an SOD.

14. Synergistic products containing pentoxifylline, α-tocopherol and either a compound with anticytokine action or a diphosphonate as a combined preparation for simultaneous, separate or sequential use in the treatment of localized or diffuse fibrosis, or connective tissue/vascular tissue degeneration.
